(19) Europäisches Patentamt

European Patent Office

Office européen des brevets

(11) EP 1 250 885 A2

(12) **EUROPEAN PATENT APPLICATION**

(43) Date of publication:
23.10.2002 Bulletin 2002/43

(51) Int Cl.⁷: $A61B\ 5/00$

(21) Application number: 01130113.2

(22) Date of filing: 18.12.2001

(84) Designated Contracting States:
AT BE CH CY DE DK ES FI FR GB GR IE IT LI LU
MC NL PT SE TR
Designated Extension States:
AL LT LV MK RO SI

(30) Priority: 05.04.2001 JP 2001107298

(71) Applicant: Colin Corporation
Komaki-shi, Aichi-ken (JP)

(72) Inventor: Takatani, Setsuo
Tokyo 101-0062 (JP)

(74) Representative:
Winter, Brandl, Fürniss, Hübner, Röss, Kaiser,
Polte Partnerschaft
Patent- und Rechtsanwaltskanzlei
Alois-Steinecker-Strasse 22
85354 Freising (DE)

(54) **Oxygen-saturation measuring apparatus**

(57)  An apparatus (8) for measuring an oxygen saturation of a living subject, comprising a light source (26) which emits, toward a tissue (14) of the subject, a first light having a first wavelength whose absorption coefficient with respect to hemoglobin changes depending upon oxygen saturation, and a second light having a second wavelength whose absorption coefficient with respect to hemoglobin does not change depending upon oxygen saturation; a light sensor (28) which detects a secondary light resulting from the first light, and a secondary light resulting from the second light, and produces a first light signal representing the detected secondary light of the first light, and a second light signal representing the detected secondary light of the second light; and an oxygen-saturation determining means (64, S3) for determining the oxygen saturation of the subject, based on a ratio of a magnitude of one of the first and second light signals to a magnitude of the other of the first and second light signals, according to a predetermined linear relationship between oxygen saturation and signal-magnitude ratio. The first wavelength falls in a range of from 720 nm to 740 nm and the second wavelength falls in a range of from 800 nm to 840 nm.

FIG. 1

**Description**

BACKGROUND OF THE INVENTION

Field of the Invention

The present invention relates to an apparatus for measuring a degree of oxygen saturation of a total blood consisting of an arterial blood and a venous blood.

Related Art Statement

There is known an oxygen-saturation measuring apparatus which emits, toward tissue of a living subject via skin, a first light having a first wavelength $\lambda 1$ whose absorption coefficient with respect to hemoglobin changes depending upon oxygen saturation, and a second light having a second wavelength $\lambda 2$ whose absorption coefficient with respect to hemoglobin does not change depending upon oxygen saturation. The oxygen-saturation measuring apparatus non-invasively determines an oxygen saturation based on a ratio of one of respective intensities of respective secondary lights of the first and second lights $\lambda 1$, $\lambda 2$ to the other intensity, according to a predetermined relationship between oxygen saturation and light-intensity ratio. Here, the term "secondary light" means a backward scattering light resulting from backward scattering of an original light in the tissue, or a transmission light resulting from transmitting of an original light through the tissue. Thus, the oxygen-saturation measuring apparatus is of a reflection type in which a backward scattering light is used as the secondary light, or of a transmission type in which a transmission light is used as the secondary light.

Japanese Patent Document No. 63-92335 or its corresponding U.S. Patent No. 4,714,080 discloses an example of the reflection type oxygen-saturation measuring apparatus. This apparatus can continuously measure blood oxygen saturation of a living subject, without a blood-evacuating operation.

Meanwhile, the conventional oxygen-saturation measuring apparatus employs, as the first light $\lambda 1$, a red light having a wavelength of 660 nm, employs, as the second light $\lambda 2$, an infrared light having a wavelength of 910 nm, and employs a linear relationship as the predetermined relationship used to determine the oxygen saturation based on the ratio of one of the respective secondary lights of the first and second lights $\lambda 1$, $\lambda 2$ to the other intensity.

However, in the case where the red light having the wavelength of 660 nm and the infrared light having the wavelength of 910 nm are used as the first light $\lambda 1$ and the second light $\lambda 2$, respectively, a relationship between oxygen saturation and light-intensity ratio is, in fact, non-linear. In addition, though the above-indicated linear relationship depends on hematocrit, i.e., a proportion (%) of a volume of blood cells relative to a total volume of blood, the conventional apparatus assumes that hematocrit falls in a certain range. Therefore, the oxygen saturation measured by the conventional apparatus is not sufficiently accurate.

SUMMARY OF THE INVENTION

It is therefore an object of the present invention to provide an oxygen-saturation measuring apparatus which can measure an highly accurate oxygen saturation.

The above object has been achieved by the present invention. According to the present invention, there is provided an apparatus for measuring an oxygen saturation of a living subject, comprising a light source which emits, toward a tissue of the subject, a first light having a first wavelength whose absorption coefficient with respect to hemoglobin changes depending upon oxygen saturation, and a second light having a second wavelength whose absorption coefficient with respect to hemoglobin does not change depending upon oxygen saturation; a light sensor which detects a secondary light resulting from the first light, and a secondary light resulting from the second light, and produces a first light signal representing the detected secondary light of the first light, and a second light signal representing the detected secondary light of the second light; and an oxygen-saturation determining means for determining the oxygen saturation of the subject, based on a ratio of a magnitude of one of the first and second light signals to a magnitude of the other of the first and second light signals, according to a predetermined linear relationship between oxygen saturation and signal-magnitude ratio, wherein the first wavelength falls in a range of from 720 nm to 740 nm and the second wavelength falls in a range of from 800 nm to 840 nm.

According to the combination of the first and second wavelengths, the linearity of the relationship between oxygen saturation and the signal-magnitude ratio increases, and accordingly the accuracy of the oxygen saturation determined according to the linear relationship improves. In addition, since the first wavelength falls in the range of from 720 nm to 740 nm, the influence of change of hematocrit to the first light signal decreases and accordingly the accuracy of the oxygen saturation determined according to the linear relationship further improves.

Preferably, the oxygen-saturation measuring apparatus further comprises a hematocrit determining means for determining a hematocrit of the subject based on the magnitude of the second light signal produced by the light sensor, according to a predetermined relationship between hematocrit and signal magnitude. The second wavelength is less influenced by the oxygen saturation and, if hematocrit is determined based on the magnitude of the second light signal representing the secondary light having the second wavelength, then the determined hematocrit enjoys a high accuracy.

Preferably, the second wavelength is 805 nm. Since the absorption coefficient of the light having the wavelength of 805 nm is not influenced at all by the oxygen saturation, the hematocrit determined by the hematocrit determining means enjoys the highest accuracy.

Preferably, the oxygen-saturation measuring apparatus further comprises an amplifier which amplifies each of the first light signal and the second light signal produced by the light sensor and supplies each of the amplified first light signal and the amplified second light signal to the oxygen-saturation determining means; and a probe which is adapted to be worn on the tissue of the subject and which houses the light source, the light sensor, and the amplifier. According to this feature, the distance between the light sensor and the amplifier is minimized and accordingly noise mixed with the first and second light signals is minimized.

BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:

Fig. 1 is a view showing a construction of a reflection-type oximeter including a reflection-type probe, to which the present invention is applied;
Fig. 2 is a bottom view of the reflection-type probe shown in Fig. 1;
Fig. 3 is a cross-section view of the reflection-type probe shown in Fig. 1;
Fig. 4 is a graph showing a relationship between wavelength of light and absorption coefficient of oxygenated hemoglobin or non-oxygenated hemoglobin;
Fig. 5 is a view showing a wiring pattern provided on an amplifier-section side of a substrate of the reflection-type probe;
Fig. 6 is a view showing a wiring pattern provided on a sensor-section side of the substrate of the reflection-type substrate;
Fig. 7 is a view showing an electric circuit of the reflection-type probe;
Fig. 8 is a flow chart representing a control program according to which a control device of the reflection-type oximeter of Fig. 1 is operated;
Fig. 9 is a graph showing a plurality of curves each of which represents a relationship between hematocrit Hct and normalized intensity NI;
Fig. 10A is a graph showing a relationship between invasively measured oxygen saturation and reference light-intensity ratio R'; and
Fig. 10B is a graph showing a relationship between invasively measured oxygen saturation and light-intensity ratio R.

DETAILED DESCRIPTION OF PREFERRED EMBODIMENTS

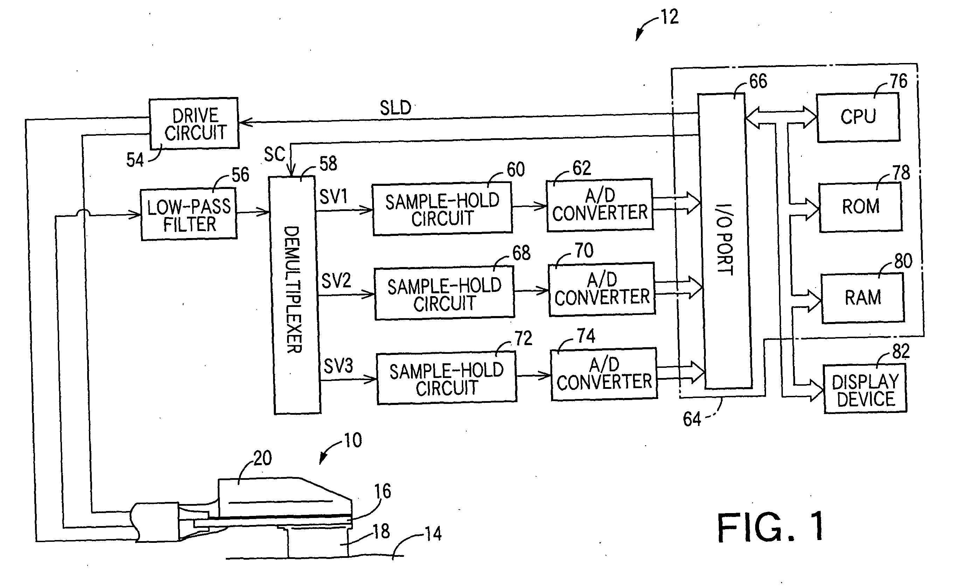

Hereinafter, there will be described a preferred embodiment of the present invention in detail by reference to the drawings. Fig. 1 shows a construction of a reflection-type oximeter 12 including a reflection-type probe 10 and functioning as a reflection-type oxygen-saturation measuring apparatus.

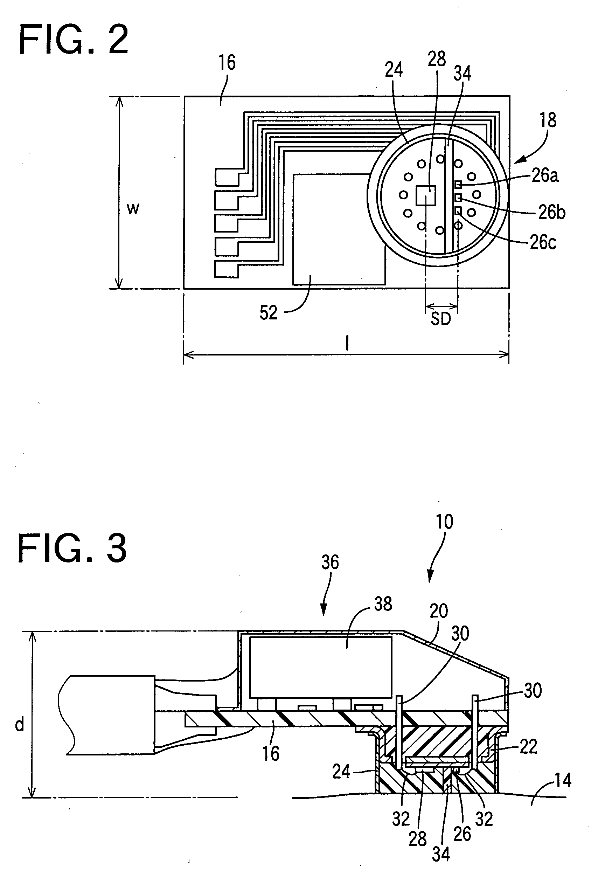

The reflection-type probe 10 functions as a probe which is adapted to be worn on a body surface 14 of a tissue (e.g., forehead or finger) of a living subject in which a considerably high density of peripheral blood vessels are present. The reflection-type probe 10 includes a substrate 16 to one of opposite surfaces of which a sensor section 18 is fixed that is adapted to be held in close contact with the body surface 14 and to the other surface of which an aluminum-based case 20 is fixed that protects an internal amplifier section 36 (Fig. 3) against external noise.

Fig. 2 is a plan view of the body-surface-14-side surface of the substrate 16 of the reflection-type probe 10; and Fig. 3 is a cross-section view of the probe 10. The reflection-type probe 10 has a considerably small size having a thickness d of about 10 mm, a length ℓ of about 21 mm, and a width w of about 12 mm. The sensor section 18 includes a considerably shallow, cylindrical, element-support member 22 having a bottom wall. An outer circumferential surface of the element-support member 22 is covered by a sensor case 24. The sensor case 24 has an axial length greater than that of the element-support member 22. A body-surface-14-side surface of the bottom wall of the element-support member 22 supports a first light-emitting element 26a, a second light-emitting element 26b, and a third light-emitting element 26c (hereinafter, referred to as the "light-emitting elements 26" when it is not needed to distinguish those elements 26a, 26b, 26c from one another). Each of the light-emitting element 26a, 26b, 26c functions as a light source. The element-support member 22 additionally supports a light-receiving element 28 functioning as a light sensor. Each of the light-emitting elements 26 is provided by an LED (light emitting diode) or the like, and the light-receiving element 28 is provided by a photodiode, a phototransistor, or the like. The first, second, and third light-emitting elements 26a, 26b, 26c are arranged on a straight line parallel to the widthwise direction of the substrate 16. A distance SD between each of the light-emitting elements 26a, 26b, 26c and the light-receiving element 28 as measured in the lengthwise direction of the substrate 16 is minimized in a physically permissible range, e.g., is selected at 1.8 mm in the present embodiment. A plurality of electrically conductive leg members 30 extend from the bottom wall of the element-support member 22, toward the substrate 16, and the light-emitting elements 26 and the light-receiving element 28 are connected to the leg members 30, respectively, via respective electric wires 32.

A light-blocking wall 34 is for preventing the lights emitted toward the body surface 14 by the light-emitting elements 26 and directly reflected from the body surface 14, from being received by the light-receiving element 28. The light-blocking wall 34 extends from the bottom wall of the element-support member 22 toward the body surface 14 in a direction parallel to the axial direction of the sensor case 24, and an end surface of the light-blocking wall 34 is aligned with an open end of the sensor case 24. A transparent resin fills vacant spaces left between the open end of the sensor case 24 and the bottom wall of the element-support member 22, so as to protect the light-emitting elements 26 and the light-receiving element 28, and another transparent resin fills vacant spaced left between the bottom wall of the element-support member 22 and the substrate 16.

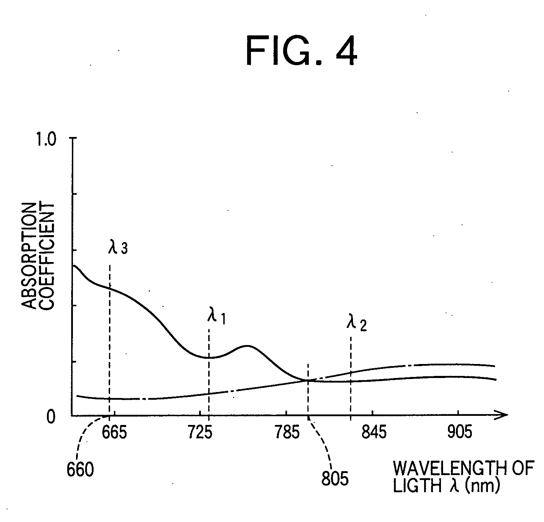

The first light-emitting element 26a emits a first light having a first wavelength $\lambda 1$ of 730 nm; the second light-emitting element 26b emits a second light having a second wavelength $\lambda 2$ of 830 nm; and the third light-emitting element 26c emits a third light having a third wavelength $\lambda 3$ of 660 nm. Fig. 4 is a graph showing a relationship between wavelength of light and absorption coefficient of oxygenated hemoglobin, indicated at one-dot chain line, or non-oxygenated hemoglobin, indicated at solid line. As can be understood from Fig. 4, the first wavelength $\lambda 1$ exhibits a considerably great difference between respective absorption coefficients with respect to oxygenated hemoglobin and non-oxygenated hemoglobin. Thus, it can be said that the first wavelength $\lambda 1$ is a wavelength whose absorption coefficient with respect to hemoglobin changes depending upon oxygen saturation. On the other hand, the second wavelength $\lambda 2$ exhibits substantially equal absorption coefficients with respect to oxygenated hemoglobin and non-oxygenated hemoglobin, and it can be said that the second wavelength $\lambda 2$ is a wavelength whose absorption coefficient with respect to hemoglobin does not change depending upon oxygen saturation. The third wavelength $\lambda 3$ exhibits a greater difference between respective absorption coefficients with respect to oxygenated hemoglobin and non-oxygenated hemoglobin, than the difference exhibited by the first wavelength $\lambda 1$, and it is the reason why the wavelength $\lambda 3$ has conventionally been used as a wavelength whose absorption coefficient with respect to hemoglobin changes with oxygen saturation. The third wavelength $\lambda 3$ is just a reference wavelength that is employed to show the advantages obtained by using 730 nm as the first wavelength $\lambda 1$.

As shown in Fig. 4, the difference between the respective absorption coefficients exhibited by the third wavelength $\lambda 3$ with respect to oxygenated hemoglobin and non-oxygenated hemoglobin is as about two times great as that exhibited by the first wavelength $\lambda 1$. Thus, the third wavelength $\lambda 3$ exhibits a higher sensitivity to oxygen saturation SO2 than that exhibited by the first wavelength $\lambda 1$. However, the third wavelength $\lambda 3$ also exhibits a higher sensitivity to hematocrit Hct than that exhibited by the first wavelength $\lambda 1$. Therefore, in the case where the third wavelength $\lambda 3$ is employed, the oxygen saturation SO2 changes by change of the hematocrit Hct. On the other hand, though the first wavelength $\lambda 1$ exhibits a lower sensitivity to the oxygen saturation SO2 than the third wavelength $\lambda 3$, the first wavelength $\lambda 1$ also exhibits a lower sensitivity to the hematocrit Hct than the third wavelength $\lambda 3$. Thus, in the case where the first wavelength $\lambda 1$ is employed, the oxygen saturtion SO2 is less influenced by the hematocrit Hct.

As shown in Fig. 3, the substrate 16 of the reflection-type probe 10 supports the amplifier section 36 on one of opposite sides of the substrate 16 that is protected by the case 20. The amplifier section 36 includes an operational amplifier 38, a plurality of chip resistors R, and a plurality of chip capacitors C.

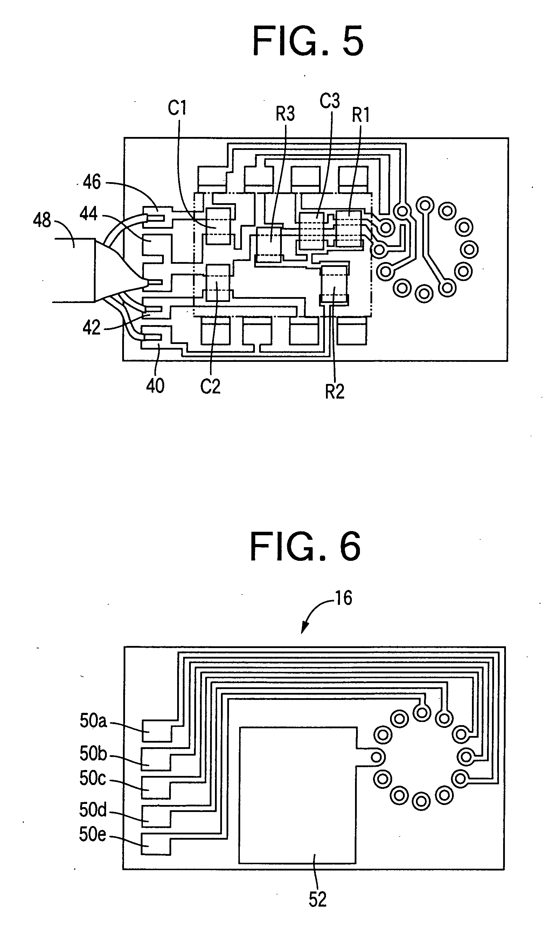

Fig. 5 shows a wiring pattern of the amplifier section 36 supported by the substrate 16. As shown in Fig. 5, the the amplifier section 36 provided on the substrate 16 includes a first chip resistor R1, a second chip resistor R2, a third chip resistor R3, a first chip capacitor C1, a second chip capacitor C2, and a third chip capacitor C3 that are fixed to respective prescribed positions on the wiring pattern. As shown in Fig. 3, the operational amplifier 38 is provided above the chip resistors R and the chip capacitors C. In addition, the wiring pattern includes, at a left-hand end thereof, an output portion (OUT) 40, a positive-potential portion (V+) 42, a ground-potential portion (GND) 44, and a negative-potential portion (V-) 46 to which appropriate core wires of a shielding wire 48 and the shielding wire 48 itself are connected, respectively.

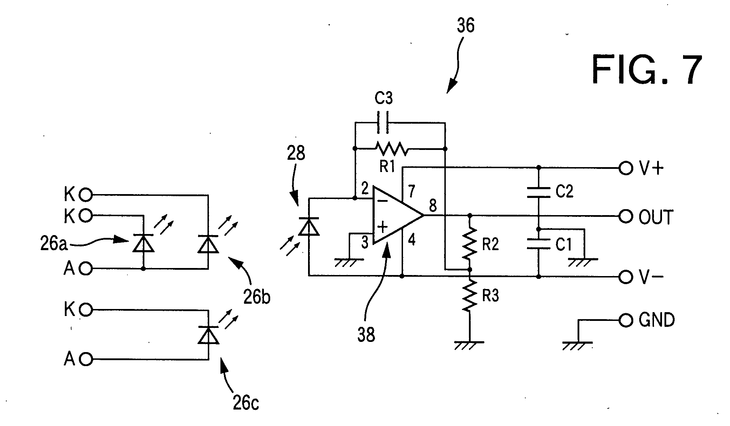

Fig. 6 shows a wiring pattern of the sensor section 18 supported by the substrate 16. As shown in Fig. 6, the wiring pattern includes, at a left-hand end thereof, five connection portions 50a, 50b, 50c, 50d, 50e to which appropriate core wires of the shielding wire 48, not shown in Fig. 6, are connected, respectively. The connection portions 50a-50e are electrically coupled, at the other end of the wiring pattern, the leg members 30 supported by the element-support member 22, respectively. Thus, the connection portion 50a is connected to a negative electrode of the first light-emitting element 26a; the connection portion 50b is connected to a negative electrode of the second light-emitting element 26b; the connection portion 50c is connected to a positive electrode of the third light-emitting element 26c; the connection portion 50d is connected to respective positive electrodes of the first and second light-emitting elements 26a, 26b; and the connection portion 50e is connected to a negative electrode of the third light-emitting element 26c. A shielding conductor 52 is fixed to a central portion of the sensor-section-18-side surface of the substrate 16. The shielding conductor 52 is, like the aluminum-based case 20, provided for blocking external noise. Fig. 7 shows an electric circuit of the reflection-type probe 10.

Back to Fig. 1, the light-emitting elements 26 (26a, 26b, 26c) of the reflection-type probe 10 sequentially emit, according to signals supplied from a drive circuit 54, the respective lights, each for a prescribed time duration, at a considerably high frequency of from several hundred hertz to several kilohertz. The lights emitted toward the body surface 14 by the light-emitting elements 26 scatter backward from the tissue of the body surface 14, and the backward scattering lights having the first wavelength $\lambda 1$, the second wavelength $\lambda 2$, and the third wavelength $\lambda 3$ are sequentially received by the light-receiving element 28. After the backward scattering lights received by the light sensor 28 are amplified by the amplifier section 36 housed in the reflection-type probe 10, a first light signal SV1 representing the backward scattering light having the first wavelength $\lambda 1$, a second light signal SV2 representing the backward scattering light having the second wavelength $\lambda 2$, and a third light signal SV3 representing the backward scattering light having the third wavelength $\lambda 3$, are supplied from the probe 10 to a low-pass filter 56. The low-pass filter 56 removes, from the light signals SV (SV1, SV2, SV3) supplied thereto, noise having frequencies higher than a frequency of a pulse wave, and supplies the noise-free light signals SV to a demultiplexer 58.

The demultiplexer 58 is switched, according to switch signals SC, described later, in synchronism with the light emissions of the light-emitting elements 26. Thus, the demultiplexer 58 successively supplies, to an I/O port 66 of a control device 64, the first light signal SV1 via a sample-and-hold circuit 60 and an A/D (analog-to-digital) converter 62, the second light signal SV2 via a sample-and-hold circuit 68 and an A/D converter 70, and the third light signal SV3 via a sample-and-hold circuit 72 and an A/D converter 74. The sample-and-hold circuits 60, 68, 72 hold the current light signals SV1, SV2, SV3 input thereto, respectively, and do not output those current signals to the A/D converters 62, 70, 74 before the prior signals SV1, SV2, SV3 are completely converted by those A/D converters, respectively.

The I/O port 66 is connected via data bus lines to a CPU (central processing unit) 76, a ROM (read only memory) 78, a RAM (random access memory) 80, and a display device 82. The CPU 76 carries out a measuring operation according to the control program pre-stored in the ROM 78, by utilizing the temporary-storage function of the RAM 80, and outputs command signals SLD to the drive circuit 54 via the I/O port 66, so that the first, second, and third light-emitting elements 26a, 26b, 26c sequentially emit the respective lights, each for a prescribed time duration, at a considerably high frequency of from several hundred hertz to several kilohertz. In synchronism with the light emissions of the first, second, and third light-emitting elements 26a, 26b, 26c, the CPU 76 additionally outputs the switch signals SC to the demultiplexer 58 to thereby switch the same 58 so that the first light signal SV1 is supplied to the sample-and-hold circuit 60, the second light signal SV2 is supplied to the sample-and-hold circuit 68, and the third light signal SV3 is supplied to the sample-and-hold circuit 72. Moreover, the CPU 76 determines, according to the control programs pre-stored in the ROM 78, an oxygen saturation S02 of a blood flowing through the peripheral blood vessels, based on respective intensities of the scattering lights represented by the first, second, and third light signals SV1, SV2, SV3, and determines a hematocrit Hct of the blood flowing through the peripheral blood vessels. In addition, the CPU 76 operates the display device 82 to display the thus determined oxygen saturation SO2 and hematocrit Hct.

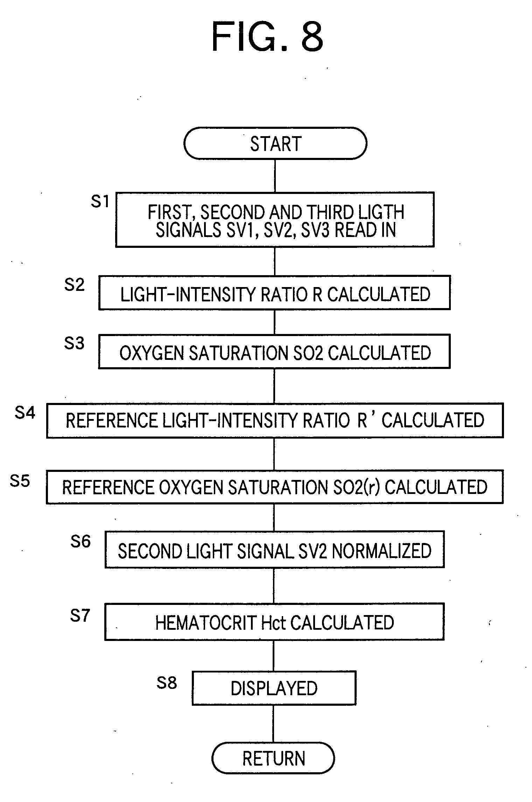

Next, there will be described a control operation of the control device 64, by reference to the flow chart of Fig. 8. First, at Step SA1 (hereinafter, "Step" is omitted, if appropriate), the control device reads in the first light signal SV1 representing the scattering light having the first wavelength $\lambda 1$, the second light signal SV2 representing the scattering light having the second wavelength $\lambda 2$, and the third light signal SV3 representing the scattering light having the third wavelength $\lambda 3$.

Then, at S2, the control device calculates a ratio R (= $I_2/I_1$) of an intensity $I_2$ of the scattering light having the second wavelength $\lambda 2$, corresponding to a magnitude or voltage of the second light signal SV2 read in at S1, to an intensity $I_1$ of the scattering light having the first wavelength $\lambda 1$, corresponding to a magnitude or voltage of the first light signal SV1 read in at S1. Subsequently, at S3 corresponding to the oxygen-saturation determining means, the control device calculates an oxygen saturation SO2 based on the light-intensity ratio R calculated at S2, according to the following Expression 1:

$$\text{(Expression 1)} \qquad SO2 = A + B \times R$$

where A, B are constants which are determined, in advance, based on experiments using blood.

Next, at S4, the control device calculates a ratio R' (= $I_2/I_3$) of the intensity $I_2$ of the scattering light having the second wavelength λ2, corresponding to the magnitude of the second light signal SV2 read in at S1, to an intensity $I_3$ of the scattering light having the third wavelength λ3, corresponding to a magnitude or voltage of the third light signal SV3 read in at S1. Hereinafter, this ratio will be referred to as the reference light-intensity ratio R'. Subsequently, at S5, the control device calculates a reference oxygen saturation SO2(r) based on the reference light-intensity ratio R' calculated at S4, according to the following Expression 2:

$$\text{(Expression 2)} \qquad SO2(r) = A + B \times R'$$

Thus, the reference oxygen saturation SO2(r) is obtained by using the third wavelength λ3 that has been used as a wavelength whose absorption coefficient with respect to hemoglobin changes depending upon oxygen saturation, and is used for comparison with the oxygen saturation SO2 obtained by using the first wavelength λ 1. The constants A, B employed in Expression 1 are also employed in Expression 2.

Next, at S6, the control device calculates a normalized intensity NI by normalizing the intensity $I_2$ of the scattering light represented by the second light signal SV2 read in at S1. This normalized intensity NI is calculated by using a reference value that is obtained, in advance, by measuring an intensity of a light scattering from, e.g., milk.

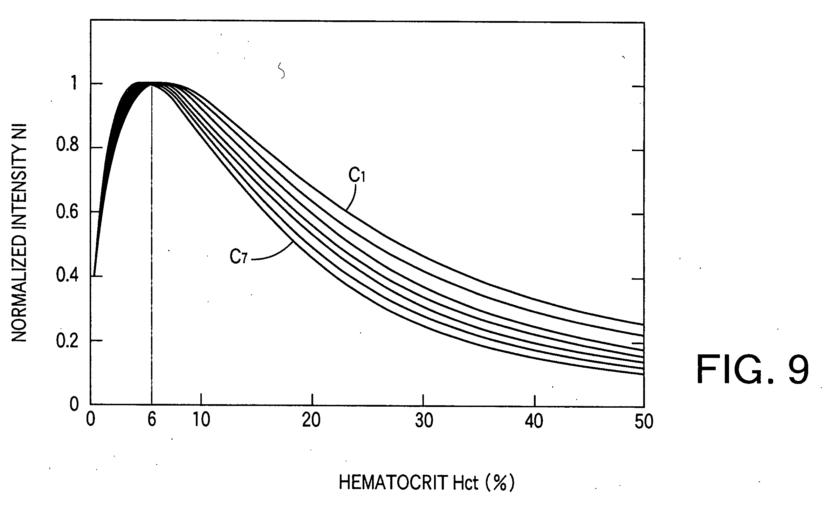

Then, at S7 corresponding to the hematocrit determining means, the control device calculates a hematocrit Hct based on the normalized intensity NI calculated at S6, according to a curve C1 shown in Fig. 9. Fig. 9 shows seven curves C1 to C7 each of which represents a relationship between hematocrit Hct and normalized intensity NI. The seven curves C1 to C7 correspond to seven different distances SD between the light-emitting elements 26a, 26b, 26c and the light-receiving element 28. The curve C1 corresponds to the smallest distance SD = 1.8 mm; and the curve C7 corresponds to the greatest distance SD = 2.4 mm. In the present embodiment, the smallest distance SD is equal to 1.8 mm, and accordingly the relationship represented by the curve C1 is employed. The reason why the smallest distance SD = 1.8 mm is employed in the reflection-type probe 10 is as follows: In a range of hematocrit Hct that is higher than 6 %, the curve C1 shows the highest linearity of all the curves C1 to C7. Therefore, it is desirable to minimize the distance SD to determine hematocrit Hct with high accuracy. Hence, the reflection-type probe 10 employs the smallest distance SD = 1.8 mm.

Then, at S8, the control device operates the display device 82 to display the oxygen saturation SO2 calculated at S3, the reference oxygen saturation SO2(r) calculated at S5, and the hematocrit Hct determined at S7. Subsequently, the control goes back to S1 and the control device repeats S1 and the following steps, so as to successively measure oxygen-saturation values SO2 and hematocrit values Hct.

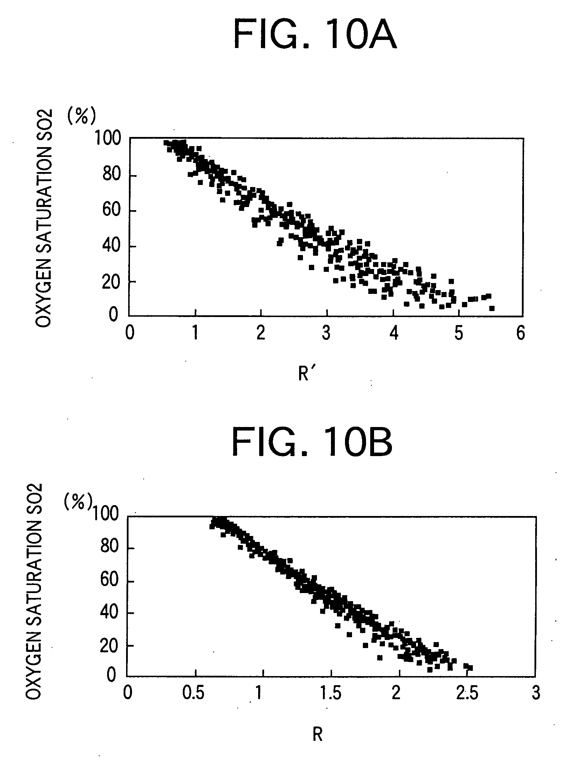

Fig. 10A is a graph showing a relationship between invasively measured oxygen saturation values and reference light-intensity ratio values R'; and Fig. 10B is a graph showing a relationship between invasively measured oxygen saturation values and light-intensity ratio values R. From those graphs, it can be seen that the light-intensity ratio values R show a distribution which can be better approximated by a straight line than a distribution of the reference light-intensity ratio values R'.

TABLE 1 shows respective RMS values calculated for the light-intensity ratio R and the reference light-intensity ratio R'. An RMS value is calculated by first determining a linear function approximating the relationship, shown in Fig. 10A or 10B, squaring a slope of the thus determined linear function, averaging respective squared slopes obtained from a number of living subjects, and determining a square root of the thus obtained average. A smaller RMS value indicates that a relationship between invasively measured oxygen saturation and reference light-intensity ratio R' or light-intensity ratio R is better approximated by a linear function. In addition, invasively measured oxygen saturation values are highly reliable. Therefore, a smaller RMS value indicates a higher reliability of the reference oxygen saturation values SO2(r) or the oxygen saturation values SO2 calculated according to Expression 1 or Expression 2. Thus, TABLE 1 shows that the oxygen saturation values S02 calculated based on the light-intensity ratio values R are more reliable than the reference oxygen saturation values SO2(r) calculated based on the reference light-intensity ratio values R'.

(TABLE 1)

|  | LIGHT-INTENSITY RATIO | REFERENCE LIGHT-INTENSITY RATIO |
|---|---|---|
| RMS | 4.24 % | 6.43 % |
| (The number, n, of the subjects is 365 in each case.) | | |

As is apparent from the foregoing description of the present embodiment, the combination of the above-described specific wavelengths increases the linearity of the relationship between oxygen saturation SO2 and light-intensity ratio R, and accordingly increases the accuracy of oxygen saturation values SO2 determined according to Expression 1. In addition, since 730 nm is used as the first wavelength $\lambda 1$, the magnitude of the first light signal SV1 is less influenced by change of the hematocrit Hct. Thus, the accuracy of the oxygen saturation values SO2 determined according to Expression 1 is improved.

In addition, in the present embodiment, the hematocrit Hct is determined based on the magnitude of the second light signal SV2 that corresponds to the intensity $I_2$ of the scattering light having the second wavelength $\lambda 2$. Therefore, the hematocrit Hct enjoys a high accuracy.

Moreover, in the present embodiment, the light-emitting elements 26, the light-receiving element 28, and the operational amplifier 38 that amplifies the signal produced by the light-receiving element 28 are also housed by the probe 10 that is adapted to be worn on the living subject. Since the distance between the light-receiving element 28 and the operational amplifier 38 is minimized, the light signals SV1, SV2, SV3 are effectively prevented from being mixed with noise.

While the present invention has been described in its preferred embodiment by reference to the drawings, it is to be understood that the invention may otherwise be embodied.

For example, in the illustrated embodiment, the first light-emitting element 26a emits the light having the first wavelength $\lambda 1$ of 730 nm. However, the first wavelength $\lambda 1$ may be any other wavelength falling in a range of from 720 nm to 740 nm, for the following reason: In the illustrated embodiment, the first light-emitting element 26a is described such that the element 26a emits the light having the wavelength of 730 nm. However, a light emitted by a common light source such as LED has frequencies ranging over a certain frequency range. Therefore, the light emitted by the first light-emitting element 26a has wavelengths ranging over a certain wavelength range whose center is 730 nm. A wavelength range defined by respective half-value widths (each corresponding to half a light intensity at the central wavelength) on both sides of the central wavelength of 730 nm is a range of from 720 nm to 740 nm. Since this wavelength range can be deemed as limits of error of the wavelength of 730 nm, the first wavelength $\lambda 1$ may take any wavelength falling in the range of from 720 nm to 740 nm.

In addition, in the illustrated embodiment, the second light-emitting element 26b is described such that the element 26b emits the light having the second wavelength $\lambda 2$ of 830 nm. However, the second wavelength $\lambda 1$ may be 805 nm. As shown in Fig. 4, with respect to the light having the wavelength of 805 nm, oxygenated hemoglobin and non-oxygenated hemoglobin exhibit an equal absorption coefficient. Therefore, in the case where 805 nm is employed as the second wavelength $\lambda 2$, the hematocrit Hct determined at Step S7 enjoys the highest accuracy. A wavelength range defined by respective half-value widths on both sides of the central wavelength of 805 nm is a range of from 800 nm to 820 nm, and this wavelength range can be deemed as limits of error of the central wavelength of 805 nm, for the same reason as explained above. Thus, the second wavelength $\lambda 2$ emitted by the second light-emitting element 26b may take any wavelength falling in the range of from 800 nm to 820 nm. In addition, as is apparent from the graph of Fig. 4, the second wavelength $\lambda 2$ emitted by the second light-emitting element 26b may take any wavelength falling in a range of from 820 nm to 840 nm.

In addition, the illustrated reflection-type oximeter 12 is for experimental purposes only, and accordingly it employs the third light-emitting element 26c, the sample-and-hold circuit 72, the A/D converter 74, etc. for determining the reference oxygen saturation SO2(r) based on the light having the third wavelength $\lambda 3$. However, it is not needed to employ any of the third light-emitting element 26c, the sample-and-hold circuit 72, the A/D converter 74, etc. in an apparatus which is actually used to measure an oxygen saturation SO2 from a patient.

Moreover, in the illustrated embodiment, the reflection-type probe 12 is employed. However, the reflection-type probe 12 may be replaced with a transmission-type probe. In the latter case, an oxygen saturation SO2 or a hematocrit Hct is determined based on an intensity or intensities of a transmission light or lights from subject's tissue in place of the intensities I of the scattering lights from the tissue of the body surface 14.

It is to be understood that the present invention may be embodied with other changes, improvements, and modifications that may occur to a person skilled in the art without departing from the spirit and scope of the invention defined in the appended claims.

**Claims**

**1.** An apparatus (12) for measuring an oxygen saturation of a living subject, comprising:

a light source (26) which emits, toward a tissue (14) of the subject, a first light having a first wavelength whose absorption coefficient with respect to hemoglobin changes depending upon oxygen saturation, and a second light having a second wavelength whose absorption coefficient with respect to hemoglobin does not change depending upon oxygen saturation;

a light sensor (28) which detects a secondary light resulting from the first light, and a secondary light resulting from the second light, and produces a first light signal representing the detected secondary light of the first light, and a second light signal representing the detected secondary light of the second light; and

an oxygen-saturation determining means (64, S3) for determining the oxygen saturation of the subject, based on a ratio of a magnitude of one of the first and second light signals to a magnitude of the other of the first and second light signals, according to a predetermined linear relationship between oxygen saturation and signal-magnitude ratio,

wherein the first wavelength falls in a range of from 720 nm to 740 nm and the second wavelength falls in a range of from 800 nm to 840 nm.

2. An apparatus according to claim 1, further comprising a hematocrit determining means (64, S7) for determining a hematocrit of the subject based on the magnitude of the second light signal produced by the light sensor, according to a predetermined relationship between hematocrit and signal magnitude.

3. An apparatus according to claim 2, wherein the second wavelength is 805 nm.

4. An apparatus according to any one of claims 1 to 3, further comprising:

an amplifier (36, 38) which amplifies each of the first light signal and the second light signal produced by the light sensor and supplies each of the amplified first light signal and the amplified second light signal to the oxygen-saturation determining means; and
a probe (10) which is adapted to be worn on the tissue (14) of the subject and which houses the light source (26), the light sensor (28), and the amplifier.

5. An apparatus according to any one of claims 1 to 4, further comprising a memory (78) which stores the predetermined linear relationship between oxygen saturation and signal-magnitude ratio.

6. An apparatus according to any one of claims 2 to 5, further comprising a memory (78) which stores the predetermined relationship between hematocrit and signal magnitude.

7. An apparatus according to any one of claims 4 to 6, further comprising a normalizing means (64, S6) for normalizing the magnitude of the second light signal produced by the light sensor (26), and wherein the hematocrit determining means (64, S7) determines the hematocrit of the subject based on the normalized magnitude of the second light signal according to the predetermined relationship between hematocrit and signal magnitude.

8. An apparatus according to any one of claims 4 to 7, wherein the probe (10) houses the light source (26) and the light sensor (28), such that a distance between the light source and the light sensor falls in a range of 1.8 mm to 2.4 mm.

9. An apparatus according to any one of claims 4 to 8, wherein the probe (10) comprises a light-blocking wall (34) which is provided between the light source (26) and the light sensor (28).

10. An apparatus according to any one of claims 1 to 9, further comprising a display device (82) which displays the oxygen saturation determined by the oxygen-saturation determining means (64, S3).

11. An apparatus according to any one of claims 4 to 10, further comprising a display device (82) which displays the oxygen saturation determined by the oxygen-saturation determining means (64, S3) and the hematocrit determined by the hematocrit determining means (64, S7).

FIG. 1

EP 1 250 885 A2

# FIG. 2

# FIG. 3

# FIG. 4

# FIG. 5

# FIG. 6

FIG. 7

# FIG. 8

```
              ┌─────────────────┐
              │      START      │
              └─────────────────┘
                       │
  S1 ┌─────────────────────────────────────┐
     │  FIRST, SECOND AND THIRD LIGTH      │
     │  SIGNALS SV1, SV2, SV3 READ IN      │
     └─────────────────────────────────────┘
                       │
  S2 ┌─────────────────────────────────────┐
     │   LIGHT-INTENSITY RATIO R CALCULATED │
     └─────────────────────────────────────┘
                       │
  S3 ┌─────────────────────────────────────┐
     │  OXYGEN SATURATION SO2 CALCULATED   │
     └─────────────────────────────────────┘
                       │
  S4 ┌─────────────────────────────────────────┐
     │ REFERENCE LIGHT-INTENSITY RATIO R' CALCULATED │
     └─────────────────────────────────────────┘
                       │
  S5 ┌─────────────────────────────────────────┐
     │ REFERENCE OXYGEN SATURATION SO2(r) CALCULATED │
     └─────────────────────────────────────────┘
                       │
  S6 ┌─────────────────────────────────────┐
     │  SECOND LIGHT SIGNAL SV2 NORMALIZED │
     └─────────────────────────────────────┘
                       │
  S7 ┌─────────────────────────────────────┐
     │    HEMATOCRIT Hct CALCULATED        │
     └─────────────────────────────────────┘
                       │
  S8 ┌─────────────────┐
     │    DISPLAYED     │
     └─────────────────┘
                       │
              ┌─────────────────┐
              │     RETURN       │
              └─────────────────┘
```

FIG. 9

# FIG. 10A

# FIG. 10B